Europäisches Patentamt

European Patent Office

Office européen des brevets

(19)

(11) Veröffentlichungsnummer: **0 096 325**
**A2**

(12) **EUROPÄISCHE PATENTANMELDUNG**

(21) Anmeldenummer: **83105317.8**

(22) Anmeldetag: **30.05.83**

(51) Int. Cl.³: **A 61 B 3/06**

(30) Priorität: **08.06.82 US 386434**

(43) Veröffentlichungstag der Anmeldung:
**21.12.83 Patentblatt 83/51**

(84) Benannte Vertragsstaaten:
**AT BE CH DE FR GB IT LI LU NL SE**

(71) Anmelder: **Interzeag AG**
**Rietbachstrasse 5**
**CH-8952 Schlieren(CH)**

(72) Erfinder: **Hirsch, Joy**
**318 Elm Street, Apt. 1A**
**New Haven Connecticut 06511(US)**

(74) Vertreter: **Fillinger, Peter, Dr.**
**Rütistrasse 1a**
**CH-5400 Baden(CH)**

(54) Verfahren und Vorrichtung zum Bestimmen der Kontrastempfindlichkeit des menschlichen Auges.

(57) Zum Ermitteln der Kontrastempfindlichkeit eines Auges einer Versuchsperson werden dieser eine Anzahl unterschiedlicher Testmuster auf einer Bildfläche dargeboten, wobei die Testmuster ein Feld mit konstanter und ein Feld mit variabler Leuchtdichte aufweisen. Die Leuchtdichte des variablen Feldes kann nach einer ersten Art der Untersuchungsdurchführung von einem Computer vorher festgelegt oder nach einer zweiten Art mit einer Regelung durch die Versuchsperson eingestellt werden. Nach der ersten Art lässt der Computer auf dem Bildschirm eine Anzahl von Testmustern mit unterschiedlicher Schwellenbreite und unterschiedlichem Kontrast erscheinen und die Versuchsperson muss anzeigen, ob sie eine Schwelle sehen kann oder nicht. Nach der zweiten Art präsentiert der Computer eine Anzahl von Testmustern unterschiedlicher Schwellenbreiten und die Versuchsperson muss die Leuchtdichte des variablen Feldes bis zu einem Punkt regeln, an dem die Schwelle zwischen beiden Feldern verschwindet. In beiden Arten der Durchführung der Untersuchung wird die Stärke des Kontrastes gemessen, die erforderlich ist, damit die Versuchsperson eine Schwelle erkennen kann. Die Antworten der Versuchsperson werden bei beiden Arten ermittelt und mit Kontrollwerten verglichen.

FIG.3A

FIG.3C

Verfahren und Vorrichtung zum Bestimmen der Kontrastempfindlichkeit des menschlichen Auges

---

Die vorliegende Erfindung bezieht sich auf ein
Verfahren und eine Vorrichtung zum Prüfen der visuellen
Wahrnehmungsfähigkeit einer Versuchsperson für eine Schwelle zwischen benachbarten Feldern unterschiedlicher Leuchtdichte.

In den letzten Jahren hat man erkannt, dass
ein Verlust des Sehvermögens ein erstes Anzeichen für
eine Augenkrankheit ist. Somit erweist sich ein guter und
ordnungsgemäss ausgeführter Test des Sehvermögens als
wertvolles Hilfsmittel für den Augenoptiker oder Augenarzt.

Die Perimetrie ist eine der Möglichkeiten,
mit der man das Sehvermögen testen und die Funktionsfähigkeit der Netzhaut sowie das entsprechende Nervensystem des Gehirns beurteilen kann. Bei diesem Test wird
die Fähigkeit des Auges gemessen, Licht an verschiedenen
Stellen der Netzhaut wahrzunehmen. Es hat sich gezeigt,
dass bei einer Beeinträchtigung der Netzhaut oder anderer Teile des Sehsystems infolge solcher Krankheiten
wie Sehnervenentzündung, Grüner Star oder diabetischer
Netzhauterkrankung, das Auge in gewissem Masse die Fähigkeit verliert, Licht wahrzunehmen. Die Perimetrie
dient dazu, die Bereiche der Netzhaut so genau wie möglich zu bestimmen, in denen die Empfindlichkeit auf die Wahrnehmung kleiner Lichtpunkte reduziert ist.

Ein Beispiel für ein derzeit auf dem Markt erhältliches Gerät zur Messung des Gesichtsfeldes ist das automatische Perimeter OCTOPUS$^{TM}$, das von der Interzeag AG, Schlieren (CH) angeboten wird. Das automatische Perimeter OCTOPUS arbeitet mit einem computergesteuerten hemispärischen Perimeter-Projektions-System, mit dem das gesamte Gesichtsfeld geprüft werden kann. Ein ähnliches Gerät ist im US-Patent Nr. 3 705 003 von Lynn und anderen beschrieben. Dieses Gerät arbeitet mit einem Computer, um die Position, die Grösse und Intensität verschiedener Teststimuli zu steuern, die in einer Cupola an vorher festgelegten Stellen im Gesichtsfeld der Versuchsperson dargeboten werden. Die Versuchsperson, die auf den Bildschirm schaut, reagiert auf jeden Stimulus mit einem Steuergerät, um damit anzuzeigen, ob Licht wahrgenommen wurde.

Die Lichtwahrnehmung ist nur eine von mehreren Funktionen des Systems Netzhaut/Gehirn. Zu diesen Funktionen gehört auch die Wahrnehmung von Mustern, Bewegungen und Farben. Wenn die Netzhaut oder ein anderer Teil des Gesichtsinns infolge von Krankheiten beeinträchtigt ist, so ist zu erwarten, dass eine oder alle dieser Grundfunktionen beeinträchtigt werden oder verloren gehen.

In der letzten Zeit hat man hauptsächlich die Mustererkennung verwendet, um den Zustand des Auges bewerten zu können. Die Fähigkeit zur Mustererkennung lässt sich dadurch bestimmen, indem man die Kontraststärke in einem Muster festlegt, die zur Erkennung des Musters durch die Versuchsperson erforderlich ist. Die Prüfung der "Kontrastempfindlichkeit" wird als nützliches Diagnosemittel zur Früherkennung betrachtet, da ein erkranktes Auge oft die Fähigkeit verliert, Muster, d.h. einen Kontrast, wahrzunehmen, bevor es die Fähigkeit verliert, kleine Lichtpunkte zu erkennen. Folglich kann eine Erkrankung des Sehsystems die Kontrastempfindlichkeit eines Menschen beeinträchtigen, bevor sie sich auf die Fähigkeit zur Wahrnehmung kleiner Lichtpunkte oder der Sehschärfe auswirkt.

Mustererkennung ist eine komplizierte Form des Sehvermögens, bei der die Informationen, die die lichtempfindlichen Sehzellen des Auges liefern, in den höher entwickelten Zentren des Gehirns zusammengefasst werden. Infolge dessen zeigt eine Störung dieser Funktion bei den Augen schon früh erste Anzeichen einer Krankheit. Durch ein Prüfen der Kontrastempfindlichkeit kann man somit genau und früh das Auftreten einer Augenkrankheit erkennen. Diese Prüfung kann als eine Art "Fenster" oder "Sonde" zur Beurteilung der Funktion des Systems Netzhaut-Gehirn betrachtet werden.

Der Musterkontrast wird durch den Unterschied in der Leuchtdichte zwischen zwei nebeneinanderliegenden hellen und dunklen Bereichen bestimmt und lässt
sich quantitativ durch Messen der Leuchtdichte des nebeneinanderliegenden hellen und dunklen Bereichs erfassen.
Die Kontrastempfindlichkeit ist ein Mass für die Stärke
des Kontrastes, den das Auge braucht, um ein Muster zu
erkennen. Während beim Perimetrieren der zur Erkennung
eines kleinen Punktes erforderliche Lichtbetrag gemessen
wird, misst man beim Testen der Kontrastempfindlichkeit
die Kontraststärke, die zur Erkennung eines einfachen
Musters erforderlich ist. Beim Prüfen der Kontrastempfindlichkeit muss der Kontrast eines bestimmten Musters
so variiert werden, dass man die Kontraststärke des Kontrastes (Kontrast am Schwellwert oder Kontrastschwellwert) erhält, der zur Erkennung des jeweiligen Musters
erforderlich ist. Der Kontrastschwellwert ist der geringste, erkennbare Kontrast und lässt sich mit einem Prüfverfahren festlegen, das dem ähnlich ist, mit dem die
Perimetriegeräte arbeiten.

Ein Beispiel für ein kommerziell erhältliches Instrument zum Messen der Kontrastempflichkeit ist
das Gerät, das von Optronix in Evanston, Illinois, angeboten wird.

Die derzeit auf dem Markt erhältlichen Instrumente zum Prüfen der Kontrastempfindlichkeit haben
eine Reihe von Nachteilen, die sich zum Teil aus den von
ihnen verwendeten Balkentest-Mustern ergeben. Die Muster, die zur Bestimmung der Empfindlichkeit einer Versuchsperson verwendet werden, bestehen aus mehreren vertikalen Balken mit abwechselnd hellen und dunklen Streifen. Die Grösse (Breite) der Streifen ändert sich von Testteil zu Testteil. Was jedoch ungeachtet der Streifengrösse gemessen wird, ist die Mindeststärke des Kontrastes, die erforderlich ist, damit die Versuchsperson das Vorhandensein des Musters korrekt bestätigen kann. Dies ist die Kontrastschwelle. Diese Muster, auch unter der Bezeichnung "Gittermuster" bekannt, bestehen alle aus Balken ohne scharfe Kanten, d.h. sie sind verschwommen. Formal ändern sich die Balken von hell zu dunkel über einen Zwischenraum gemäss einer Sinusfunktion und werden häufig als sinusförmige, räumliche Frequenz-Gitter bezeichnet. Der Begriff "räumliche Frequenz" bezieht sich auf die Grösse der Balken, d.h. dass ein grosser Balken eine hohe Ortsfrequenz hat. Die Grösseneinheiten werden ausgedrückt in der Anzahl von Zyklen (helle und dunkle Balkenteile), die auf einem Grad der Netzhaut abgebildet werden, d.h. Zyklen/Grad.

Die Geräte, die zur Erzeugung der vorgeschriebenen Balken und zur Analyse der entsprechenden Reaktionen erforderlich sind, sind äusserst kompliziert. Ausserdem sind die derzeit verwendeten Tests zeitraubend. Es hat sich gezeigt, dass die Prüfung der Augenfunktion

- 6 -

durch eine andere als eine schnelle Rasterungs-Methode für den Patienten besonders ermüdend ist. Bei Verwendung solcher kommerziell erhältlicher Methoden muss der Patient u.U. wiederholt in die Klinik kommen, damit die Tests durchgeführt werden können. Dies hat sich für viele Patienten als eine besondere Belastung erwiesen.

Ein weiteres Problem bei den kommerziell erhältlichen Instrumenten zum Testen der Kontrastempfindlichkeit liegt darin, dass man die Kontrastempfindlichkeits-Funktion nicht richtig interpretiert. Die medizinische oder biologische Bedeutung einer verschobenen Spitzenempfindlichkeit oder eines beschleunigten Verlustes der Empfindlichkeit bei grossen Balken ist z.B. nicht bekannt.

Bei den kommerziell erhältlichen Geräten zum Messen der Kontrastempfindlichkeit wird die Stärke des Kontrastes, der zur Erkennung eines Gittermusters erforderlich ist, für die unterschiedlichen Balkengrössen (räumliche Frequenzen) bestimmt. Die Ergebnisse werden so dargestellt, dass der Kontrast oder die Schwelle (ausgedrückt in Dezibel oder Prozent) in Abhängigkeit von der räumlichen Frequenz auf der X-Achse eines Diagramms erscheint. Vergleiche mit den Normal- oder Sollwerten werden durch Punkt-zu-Punkt Subtraktionen der Werte der Versuchsperson angestellt.

Mit dieser Methode lässt sich nicht ein Gesamtindex darüber erzielen, wie gut die Funktion des Auges bei
einem Vergleich mit einer erwarteten oder Normal-Funktion
ist. Sie lässt einige mögliche Aenderungen in der Form
der Funktionskurve vermuten, d.h., dass eine Versuchsperson
bei hohen räumlichen Frequenzen eine stärkere Einbusse an
Empfindlichkeit als beispielsweise bei niedrigen räumlichen Frequenzen erlitten hat. Hierbei kann man jedoch beobachten, wie die Kontrastempfindlichkeits-Funktion sich
von der erwarteten Funktionskurve unterscheidet. Die medizinische Bedeutung ist allerdings nicht bekannt. Ausserdem ist es in vielen Fällen erforderlich, die gemessenen
Werte mit den Werten zu vergleichen, die man normalerweise von einem typischen Beobachter erwarten würde. Leider fallen solche Normal-Funktionen jeweils unterschiedlich aus. Folglich geht eine Präzision oder Genauigkeit
bei den derzeitigen Testprozeduren zur Kontrastempfindlichkeit verloren, da die Unsicherheit beim Vergleich
der Funktionskurven sehr hoch ist.

Aufgrund der obengenannten Tatsachen besteht
das Hauptziel der vorliegenden Erfindung darin, ein Verfahren und eine Vorrichtung zum Bestimmen der Kontrastempfindlichkeit des Auges einer Versuchsperson zu schaffen.

Das zweite Ziel der vorliegenden Erfindung besteht darin, ein Verfahren und eine Vorrichtung zu schaffen, mit der sich die ersten Anzeichen einer Augenkrankheit schnell erkennen lassen.

Ein drittes Ziel dieser Erfindung besteht daring, ein Verfahren und eine Vorrichtung bereitzustellen, mit dem man relativ einfach und billig ein Testmuster zum Messen der Kontrastempfindlichkeit erhalten kann.

Desweiteren soll mit der vorliegenden Erfindung ein Verfahren und eine Vorrichtung verfügbar gemacht werden, die/das schnell und einfach bei klinischen Patienten verwendet werden kann.

Ausserdem soll die vorliegende Erfindung ein Verfahren und eine Vorrichtung bereitstellen, mit der/dem die gemessenen Werte der Kontrastempfindlichkeit mühelos mit den Werten eines normalen Auges verglichen werden können.

Schliesslich besteht das Ziel der vorliegenden Erfindung darin, ein Verfahren und eine Vorrichtung zu schaffen, mit der/dem die Testergebnisse mühelos quantitativ mit den Testergebnissen einer Kontrollgruppe verglichen werden können.

Um die vorgenannten Ziele erreichen und die Vorteile wahrnehmen zu können, schlägt die Erfindung ein Verfahren bzw. eine Vorrichtung gemäss den Ansprüchen 1 bzw. 12 vor. Damit wird nicht die Kontraststärke festgelegt, die zur Erkennung vieler Balkengitter unterschiedlicher Grösse (räumliche Frequenzen) erforderlich ist, sondern die Konstraststärke, die man braucht, um eine Schwelle zu erkennen (Kontrast am Schwellwert oder Kontrastschwellwert). Der Test wird nachfolgend als "Edgetest" bezeichnet. Die sich folgenden Testmuster werden
von einem Computer vorgewählt, um eine Anzahl unterschiedlicher Schwellenbreiten darzustellen. In einigen Fällen
ist die Schwelle ein scharfer, und in anderen ein allmählicher Uebergang. Bei jedem Testmuster wird die für
den Patienten zur Erkennung einer Schwelle erforderliche Kontraststärke durch ein Verfahren festgelegt, bei
dem der Kontrast solange erhöht und vermindert wird,
bis ein Schwellwert hinsichtlich des Kontrastes feststellbar ist.

Bei einer bevorzugten Durchführungsart werden
die Testmuster elektronisch auf einem Bildschirm angezeigt, auf dem ein Feld mit konstanter oder fester
Leuchtdichte und ein Feld mit variabler Leuchtdichte
erscheint. Die Breiten der Schwellen zwischen beiden
benachbarten Feldern und der Leuchtdichtepegel des variablen Feldes werden vom Computer gesteuert und der
Versuchsperson dargeboten. Die Versuchsperson muss auf
solche Muster hinweisen, in denen sie eine Schwelle
zwischen beiden Feldern erkennt. In einer anderen
Durchführungsart wird der Helligkeitspegel des variablen Feldes von der Versuchsperson eingestellt. Hierbei
bedient die Versuchsperson ein Kontrollgerät für jedes
Schwellenbreiten-Testmuster, das der Computer auf dem
Bildschirm erscheinen lässt. Bei jedem Testmuster wird
die Versuchsperson aufgefordert, die Leuchtdichte des variablen Feldes auf einen Punkt einzustellen, an dem die Schwelle zwischen beiden Feldern verschwindet. Die Fähigkeit der Versuchsperson, das variable Feld richtig einzustellen, wird vom Computer überwacht und über eine Kontrolleinrichtung verglichen. Ungeachtet der zur Bestimmung des Kontrastschwellwertes gewählten Durchführungsart werden die Werte als Funktionskurve dargestellt, wobei der Kontrastschwellwert (Dezibel) in Abhängigkeit von der Schwellenbreite (Grade auf der Netzhaut der Versuchsperson) aufgezeichnet wird. Die Funktion der Werte einer Versuchsperson werden dann mit einer Normalfunktion verglichen. Es ergibt sich ein Index, der anzeigt, welche Unterschiede zwischen der Funktion der Versuchsperson und der Normalfunktion bestehen. Somit kann der Untersuchende an einer einzigen Zahl erkennen, ob der Sehsinn der Versuchsperson beeinträchtigt oder normal ist. Bei einer Beeinträchtigung zeigt die Zahl auch das Ausmass des Verlustes der Kontrastempfindlichkeit an.

Anhand der beiliegenden schematischen Zeichnung wird die Erfindung beispielsweise beschrieben. Es zeigen:

Fig. 1 ein repräsentatives Beispiel für die typischen Balkentest-Muster, die beim gegenwärtigen Stand der Technik zum Prüfen der Kontrastempfindlichkeit verwendet werden,

Fig. 2 eine repräsentative, dem gegenwärtigen Stand der Technik entsprechende Kontrastempfindlichkeits-Funktionskurve, bei der die Kontrastschwelle (Y-Achse) in Abhängigkeit von der Balkengrösse des Testmusters (X-Achse) dargestellt ist,

Fig.3A ein erstes Muster zum Prüfen der Kontrastempfindlichkeit, wobei das helle Feld links und das dunkle Feld rechts steht. Der Uebergang (Schwelle) zwischen den beiden Feldern ist scharf, d.h., er tritt so plötzlich auf, wie es in der Treppenfunktion auf der rechten Seite dargestellt ist,

Fig.3B ein zweites Muster zum Prüfen der Kontrastempfindlichkeit wobei der Uebergang zwischen den beiden benachbarten Feldern unterschiedlicher Leuchtdichte allmählicher erfolgt, was die Darstellung auf der rechten Seite zeigt,

Fig.3C ein drittes Muster zum Prüfen der Kontrastempfindlichkeit wobei eine ausgesprochen breite Uebergangszone (Schwelle) zwischen den beiden benachbarten Feldern unterschiedlicher Leuchtdichte besteht, was das Diagramm an der rechten Seite verdeutlicht,

Fig. 4 zeigt graphisch die Durchführung des Verfahrens,

Fig. 5 eine graphische Darstellung der Kontrastempfindlichkeit in Abhängigkeit der Schwellenbreite,

Fig. 6 ein Blockschema einer Vorrichtung zur Erzeugung von "Edgetest"-Mustern und zur Durchführung des Verfahrens,

Fig. 7 eine Alternative zur mechanischen Erzeugung und
Darbietung von Testmustern,

Fig. 8 bis 10 Beispiele für die Kontrastempfindlichkeit
verschiedener Personen im Vergleich mit
Normalpersonen.

Wie schon zuvor dargelegt wurde, kann Kontrast als der Unterschied in der Leuchtdichte zwischen zwei benachbarten Bereichen mit hoher und niedriger Helligkeit definiert werden. Kontrastempfindlichkeit ist das Mass der Stärke an Kontrast, die eine Versuchsperson braucht, um ein bestimmtes Testmuster zu sehen. Bei den dem gegenwärtigen Stand der Technik entsprechenden Messungen zur Kontrastempfindlichkeit wurde ein Testverfahren angewandt, in dem der Kontrast eines bestimmten räumlichen Frequenz-Musters variiert wurde, um die Mindeststärke an Kontrast zu bestimmen, die eine Person braucht, um ein Muster zu erkennen. Aehnlich wurden die Konstrastschwellen für räumliche Frequenz-Muster mit Balkenmustern unterschiedlicher Grösse bestimmt.

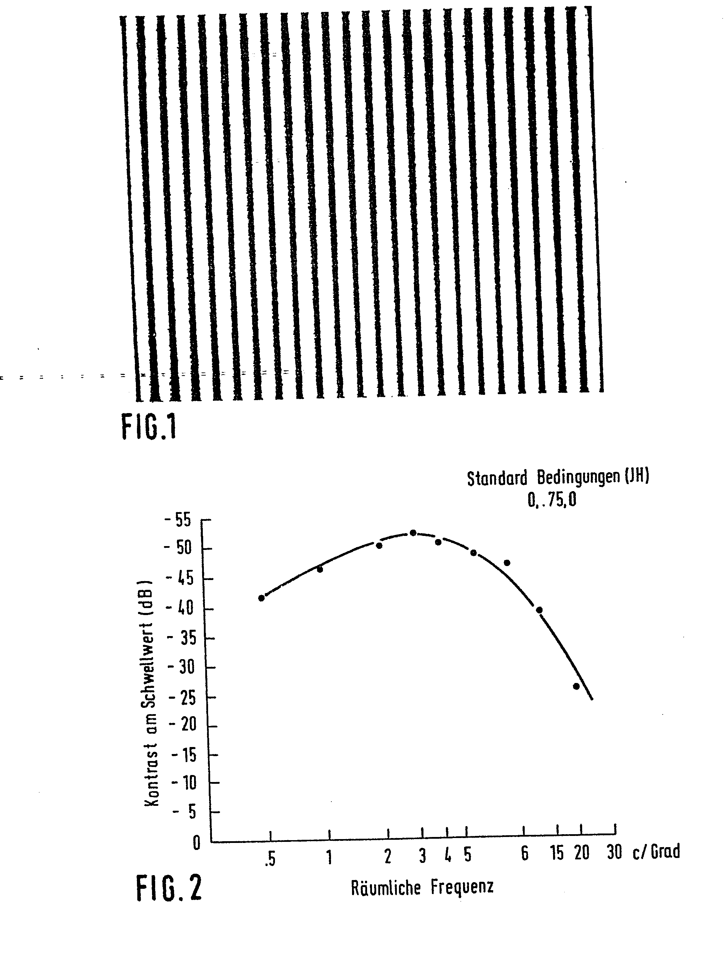

Das Muster in der Fig. 1 verdeutlicht die dem gegenwärtigen Stand der Technik entsprechenden Typen von Balkentest-Mustern, die zur Messung der Kontrastempfindlichkeit verwendet wurden. Sie hatten normalerweise die Form von Balken unterschiedlicher Grössen (Breiten).

Die Fig. 2 zeigt eine Kontrastschwellen solcher Testmuster, d.h., die geringste Stärke an Kontrast, die zur Erkennung eines Musters erforderlich ist. Sie sind in Abhängigkeit vom Muster oder der Balkengrösse dargestellt. Die Funktionen der Kontrastempfindlichkeit sind ein wichtiger Beitrag zur wissenschaftlichen Erklärung, wie das Netz/Auge/Gehirn visuelle Information kodiert. Sie werden in der Klinik dazu verwendet, die Sehfunktionen in einem kranken Auge zu prüfen.

Ein wesentlicher Nachteil des in der Fig. 1 dargestellten und dem gegenwärtigen Stand der Technik entsprechenden Balkentest-Muster besteht darin, dass die zur Erzeugung solcher Muster und zum Variieren des entsprechenden Kontrastes erforderlichen Geräte, mit denen sich der Kontrastschwellwert bestimmen lässt, äusserst kompliziert und im allgemeinen auf dem Markt nicht erhältlich sind. Demzufolge sind solche Techniken für Kliniken nicht gut geeignet. Ausserdem enthält der in solchen Verfahren verwendete und "räumliches Frequenz-Gitter" genannte Stimulus unnötige und komplizierte Merkmale.

Die Testmethode der vorliegenden Erfindung beseitigt (wenn auch nicht alle) wesentlichen Nachteile der dem gegenwärtigen Stand der Technik entsprechenden Testmuster insofern, als die Kontrastempfindlichkeit an einer einfachen Schwelle gemessen wird. Die Methode der vorliegenden Erfindung, genannt "Edgetest", bestimmt die Kontrastempfindlichkeit, die sich aus dem Unterschied in der Leuchtdichte zwischen zwei benachbarten Feldern ergibt.

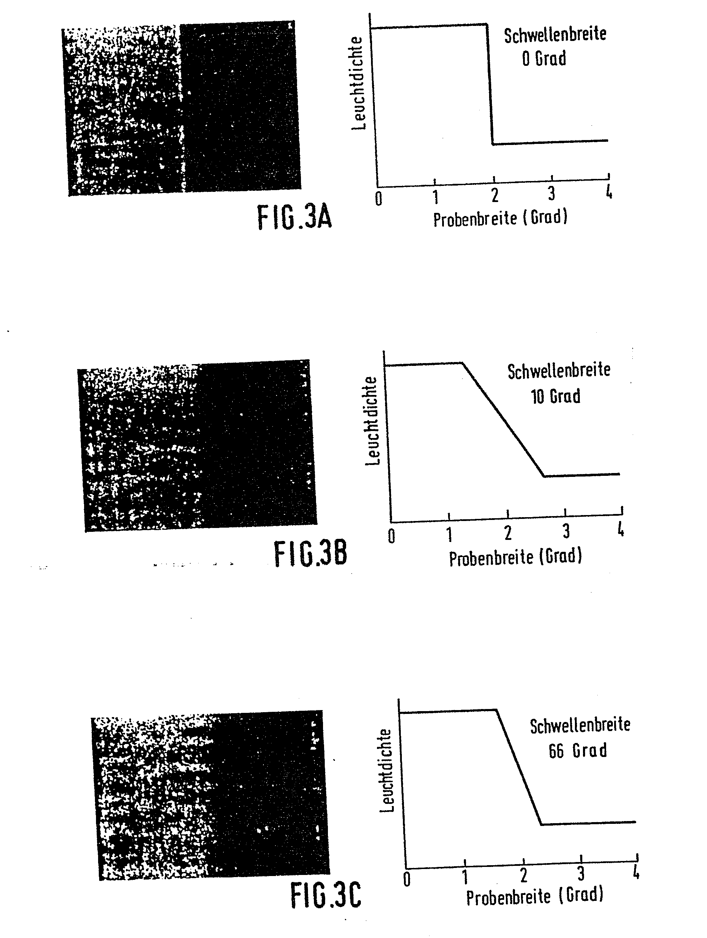

Die Leuchtdichte-Profile an der rechten Seite in den Abbildungen 3A, 3B und 3C zeigen schematisch die normalen Typen der "Edgetest"-Muster. Die Muster variieren hinsichtlich der Grösse des Uebergangs von einem Leuchtfeld zum anderen. Die Figuren an der linken Seite geben die tatsächlichen Muster wieder und sind Fotografien der entsprechenden Bildschirmanzeigen, die die "Edgetest"-Muster so zeigen, wie sie der Versuchsperson erscheinen.

In der Fig. 3A mit der Uebergangsgrösse $0^{\circ}$

besteht ein scharfer Uebergang zwischen den benachbarten Feldern. Die Fig. 3B zeigt einen graduelleren Uebergang von dem hellen zum dunklen Feld, was durch die Uebergangsgrösse von $0.66^{\circ}$ bewiesen wird. In der Fig. 3C verläuft der Uebergang zwischen dem hellen und dem dunklen Feld noch gradueller. Beweis hierfür ist die Uebergangsgrösse von $1.0^{\circ}$. Es ist ersichtlich, dass das Testmuster der Fig. 3A einen gut definierten Schwellenstimulus enthält, d.h., dass der Uebergang der Leuchtdichte vom hellen zum dunklen Feld scharf ist und somit einen optimalen Stimulus für das Auge bietet.

Die Uebergangskante in dem Testmuster der Fig. 3B ist von der Versuchsperson etwas schwieriger wahrzunehmen, und zwar wegen ihrer erhöhten Uebergangsgrösse, d.h., 0.66°. Noch schwerer ist es, das Testmuster der Fig. 3C zu erkennen, da die Uebergangskante zwischen den benachbarten Helligkeits-Feldern noch grösser ist, d.h., sie hat eine Uebergangsgrösse von 1.0°.

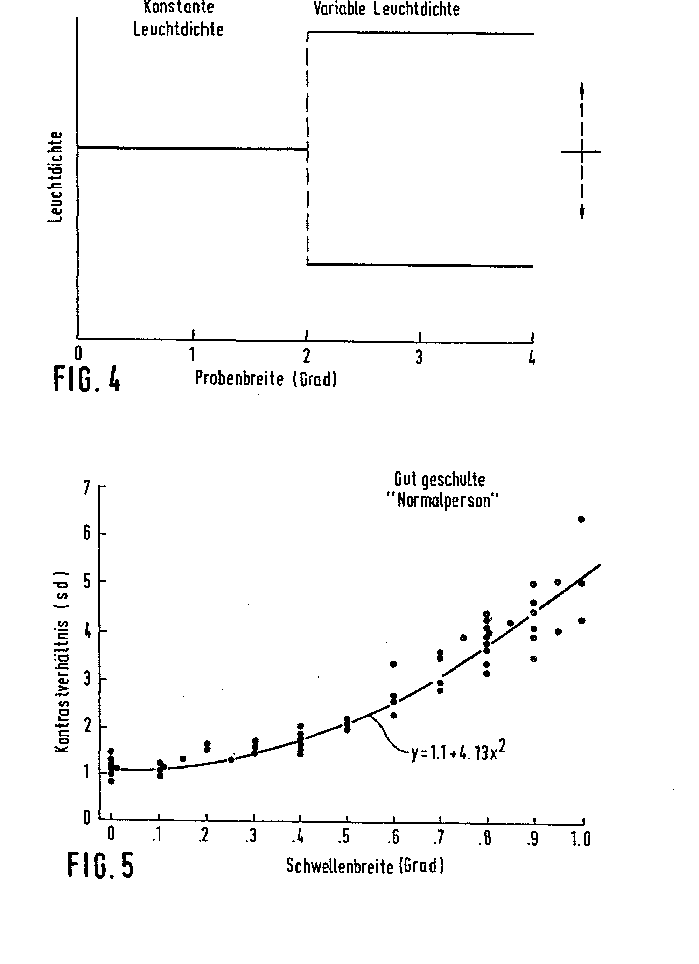

Die Fig. 4 zeigt das Testverfahren der "Edgetest"-Methode der vorliegenden Erfindung. Die Versuchsperson muss wie bei allen dargestellten Testmustern sagen, ob sie die Schwelle wahrnimmt oder nicht. Diesbezüglich ist der Test ähnlich einem Test zur Messung des Gesichtsfeldes, bei dem die Versuchsperson sagen muss, ob sie Licht wahrnimmt oder nicht.

Das Testverfahren der vorliegenden Erfindung kann geändert werden. In einem Falle wird die Versuchsperson mit den vom elektronisch auf dem Bildschirm angezeigten Testmustern konfrontiert.

Der Bildschirm ist in zwei Felder unterteilt. Ein Feld hat eine konstante oder feste Leuchtdichte, während die Leuchtdichte des anderen Feldes über oder unter der Leuchtdichte des konstanten Feldes variiert werden kann. Dies zeigt z.B. die Fig. 4, in der die Hälfte des Bildschirms, die mit "fester Seite" gekennzeichnet ist, eine konstante Leuchtdichte aufzeigt, während die Leuchtdichte des anderen Feldes über und unter die Leuchtdichte der festen Seite eingestellt werden kann.

Wie noch eingehender beschrieben werden soll, kann der "Edgetest" der vorliegenden Erfindung auf unterschiedliche Art und Weise ausgeführt werden. Die Versuchs-

- 16 -

person kann beispielsweise mit einer Reihe von Zufalls-Testmustern konfrontiert werden, indem die Schwellenbreite und die Leuchtdichte des variablen Feldes vom Computer gesteuert werden. Die Versuchsperson müsste nun nach jeder Darstellung eines Musters sagen, ob sie einen Kontrast wahrgenommen hat oder nicht. Der Computer könnte aber auch ein bestimmtes Testmuster wählen und die Versuchsperson anschliessend die Leuchtdichte des variablen Feldes mit einer Regelung so einstellen, dass die Schwelle entweder verschwindet oder erneut erscheint.

Bei der Testmethode der vorliegenden Erfindung ist vorgesehen, dass eine Gruppe normaler Versuchspersonen getestet wird und ihre Reaktionen zur Kontrolle mit allen weiteren Versuchspersonen verglichen werden. In der Fig. 5 ist die Kontrastempfindlichkeit jeder Person einer Kontrollgruppe normaler Beobachter in Abhängigkeit von der Schwellenbreite dargestellt. Zur Verdeutlichung entspricht die Schwellenbreite von 0° dem Testmuster der Fig. 3A, die Schwellenbreite von 0.66° dem Testmuster in der Fig. 3B und die Schwellenbreite von 1.0° dem noch feineren Testmuster in der Fig. 3C.

Wie man natürlich sieht, wird es für den Beobachter immer schwieriger, die Schwellenbreite zu erkennen. Diese Beziehung lässt sich durch folgende Formel quantifizieren:

$$y = 1,1 + 4,13x + Fehler,$$

wobei x die Schwellenbreite in Graden eines Gesichtswinkels, verlängert vom Auge der Versuchsperson, und y die
Messgrösse der Stärke an Kontrast darstellt, die zur Erkennung der Schwelle erforderlich ist. Diese Beziehung
wurde aus den Daten, die man mit normalen Versuchspersonen gewonnen hatte, hergestellt. Es hat sich erwiesen,
dass diese Beziehung bei allen normalen Beobachtern relativ konstant ist. Daher ist anders als bei den herkömmlichen oder bekannten Funktionen zum Testen der Kontrastempfindlichekit, die dem gegenwärtigen Stand der Technik
entsprechen, die "Edgetest"-Funktion der vorliegenden Erfindung stabiler bei den unterschiedlichen Beobachtern.

Das Verfahren nach dem der "Edgetest" ausgeführt wird, lässt sich variieren. Wichtig ist jedoch,
dass die Ergebnisse des Testes automatisch angezeigt
und mit den Ergebnissen der Kontrollgruppe verglichen
werden.

Die Testmuster sind der Versuchsperson vorzugsweise elektronisch über einen Stimulus-Bildschirm zu präsentieren. Eine Seite oder ein Feld des Bildschirms ist
auf eine konstante Leuchtigkeitsdichte gesetzt, während
die Leuchtdichte des anderen Feldes, wie schon früher im
Zusammenhang mit der Fig. 4 beschrieben wurde, variabel
gestaltet wird. Die Schwelle wird durch die Helligkeits-
Unterschiede zwischen den benachbarten Feldern erzeugt.
Der Computer wählt willkürlich eine Anzahl unterschiedlicher Testmuster und lässt sie auf dem Bildschirm erscheinen. Diese Muster enthalten Schwellen unterschiedlicher
Breiten sowie unterschiedlicher Kontraste bei jeder
Schwellenbreite.

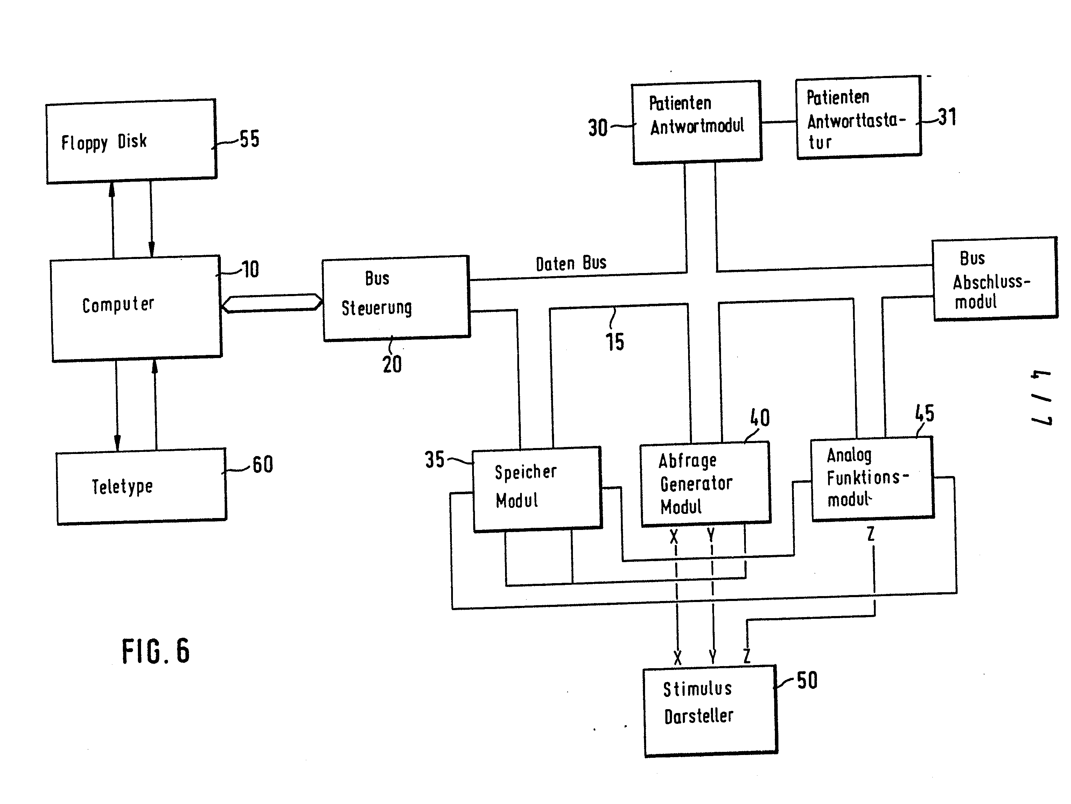

Derzeit wird ein computergesteuertes System
bevorzugt. Dieses System, bestehend aus einer programmierbaren Steuereinheit, Funktions-Modulen und Software, ist
hochautomatisiert, um die Daten noch wirksamer und objektiver zu erfassen. Dieses System ist in der Fig. 6 schematisch dargestellt.

Das System wird von einem Computer 10 gesteuert,
der nicht all zu gross sein darf und mit mässiger Geschwindigkeit arbeiten muss. Dazu gehören Massenspeicherplatz,
eine Echtzeituhr sowie ein Echtzeit-Betriebssystem und
eine Möglichkeit zur Kommunikation mit dem Bedienungspersonal.

Der "Edgetest" wird durch ein Programm im
Computer 10 ausgeführt, der sowohl mit dem Bedienungspersonal als auch dem Beobachter kommuniziert, den Betrieb
der Labor-Einrichtungen überwacht und die gewünschten Daten für eine spätere oder sofortige Analyse erfasst.

Ein Datenbus 15 verbindet die verschiedenen
Moduln miteinander und stellt einen Kommunikationsweg zum
Computer 10 bereit. Zwei Moduln sind für den Betrieb des
Bus erforderlich. Das erste ist die Bussteuerung 20, die
den Betrieb des Datenbus 15 steuert, damit eine vom System-Computer 10 und über sein eigenes Interface eingeleitete Operation ausgeführt werden kann.

Die Hardware-Funktionen werden von Funktions-Moduln im halbautomatischen Betrieb ausgeführt. Zu den verwendbaren Funktionsmoduln gehöhren ein Patientenantwortmodul 30, ein Speichermodul 35, ein Abfrage-Generator 40 und ein Analog-Funktions-Modul-45.

Zum Patientenantwortmodul 30 gehören eine Patientenantworttastatur 31. Der Beobachter antwortet über die Tastatur 31 nach jeder Darbietung eines Musters, was vom Computer 10 festgehalten wird.

Das Speichermodul 35, das Abfrage-Generator-Modul 40 sowie das Analog-Funktions-Modul 45 sind so eingestellt, dass sie die Funktionen der Stimulus-Erzeugung ausführen können. Die zwischen ihnen bestehenden Beziehungen sind schematisch in der Fig. 6 dargestellt.

Uebergangs-Muster der Stimulus-Schwelle werden von der Bussteuerung 20 errechnet und im Speichermodul 35 gespeichert. Die Testmuster werden digital erzeugt. Daher ist eine beträchtliche Flexibilität bei der Art der zu erzeugenden Testmuster gewährleistet.

Die Testmuster für die Stimulus-Schwelle werden elektronisch erzeugt und auf einen hochauflösenden Bildschirm-Monitor 50 mit hoher Intensität dargestellt. Die Labor-Software besteht aus detaillierten Algorithmen, die von der Bussteuerung 20 und den Funktions-Moduln zu implementieren sind. Das Bedienungsper-

- 21 -

sonal, das den Test ausführt, kann interaktiv mit der Software arbeiten, um die Parameter-Optionen für den gewünschten Test zu setzen und danach den Ablauf zu überwachen. Die Software erteilt die für die Funktions-Moduln erforderlichen Befehle, übernimmt die Kommunikation mit dem Bedienungspersonal und erfasst und analysiert die sich ergebenden Daten.

Jeder einzelne Test wird von seinem eigenen Programm gesteuert. Ablauf-spezifische Optionen werden von einem Dialog-System eingegeben, das das Bedienungspersonal mit dem Teletype 60 eingibt. Nach der Eingabe der Stimulus-Parameter läuft der Test vollständig unter der Steuerung der Software ab.

Der Computer 10 wählt willkürlich eine Reihe von Testmustern mit jeweils vorher bestimmten Schwellenbreiten und Kontrasten zwischen den einzelnen Feldern aus und lässt sie auf dem Bildschirm erscheinen. Dieser Computer wählt vorzugsweise fünfzig verschiedene Testmuster unterschiedlicher Schwellenbreiten und verschiedener Kontraste zwischen den einzelnen Feldern aus und lässt sie auf dem Bildschirm erscheinen. Die Unterschiede im Kontrast werden durch Variieren der Leuchtdichte auf dem variablen Feld des Bildschirms 50 erzeugt. Bei jedem einzelnen Schwellenbreiten-Testmuster wird eine Anzahl unterschiedlicher Leuchtdichten verwendet, um die KOntrastschwelle der Versuchsperson zu bestimmen.

- 21 -

In diesem Falle muss die Versuchsperson über die Patientenantworttastatur 31 nach jedem Versuch mitteilen, ob sie
eine Schwelle erkennen kann. Die Antworten der Versuchsperson werden vom Computer 10 aufgezeichnet und analysiert.

In einem zweiten Falle wählt der Computer 10
zwar auch die Schwellenbreiten der Muster aus, die für die
Versuchsperson auf dem Bildschirm 50 dargestellt werden.
Die Versuchsperson muss jedoch über die Tastatur 31 die
Leuchtdichte des variablen Feldes so hoch oder so niedrig
einstellen, bis die Schwelle infolge einer gleichen Helligkeit der beiden Felder verschwindet. Die Reaktion der Versuchsperson wird anschliessend im Computer 10 gespeichert.
Danach erscheint das nächste Testmuster automatisch auf
dem Bildschirm.

in jedem dieser Fälle wird jede Versuchsperson bei jedem Test mit einer Vielzahl von Testmustern konfrontiert.

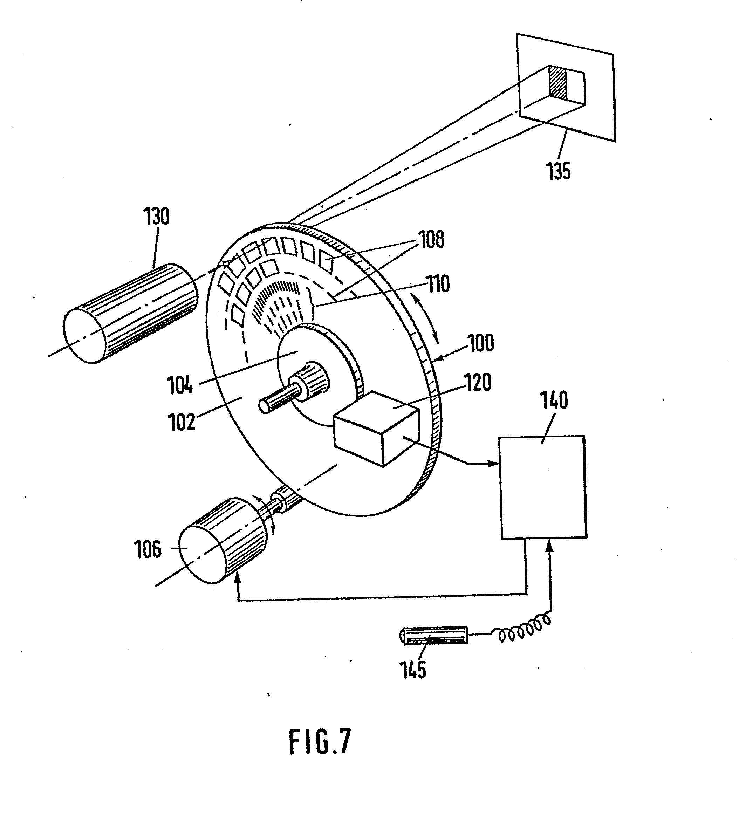

Die Testmodelle, die zur Ausführung des neuen
"Edgetestes" erforderlich sind, können auch mechanisch,
d.h. mit einem Projektor, dargestellt werden. Um das
richtige Dia schnell zu wählen und in Projektion-Position zu bringen, kann die in der Fig. 7 dargestellte
opto-mechanische Vorrichtung konstruiert werden. Ein mechanisches Projektions-System 100 ist vorgesehen. Hierzu
gehören eine Scheibe oder ein Rad 102, die/das am Umfang
eine oder mehrere Reihen Dias mit jeweils unterschiedli- chen Testmustern enthält. Jedes Dia ist mit einem Kenncode 110 gekennzeichnet. Die Scheibe 102 bewegt sich
um die Nabe 104 und wird vom Motor 106 angetrieben. Ein
an einen Computer 140 angeschlossener Codeleser 120 ist
an der Drehscheibe 102 angeschlossen, um das Dia 108
so auszurichten, dass das auf ihm enthaltene Testmuster
von der Projektorlampe 130 auf den Bildschirm 135 projiziert werden kann. Mit einer Antworttaste 145 kann die
Versuchsperson sagen, wann eine entsprechende Antwort
erforderlich ist.Ausserdem kann man mit einer oder mehreren Projektionslampen die Intensität des durch die
Dias 108 fallenden Lichtes variieren sowie andere Abänderungen vornehmen, ohne dass Sinn und Zweck der vorliegenden Erfindung entfremdet werden.

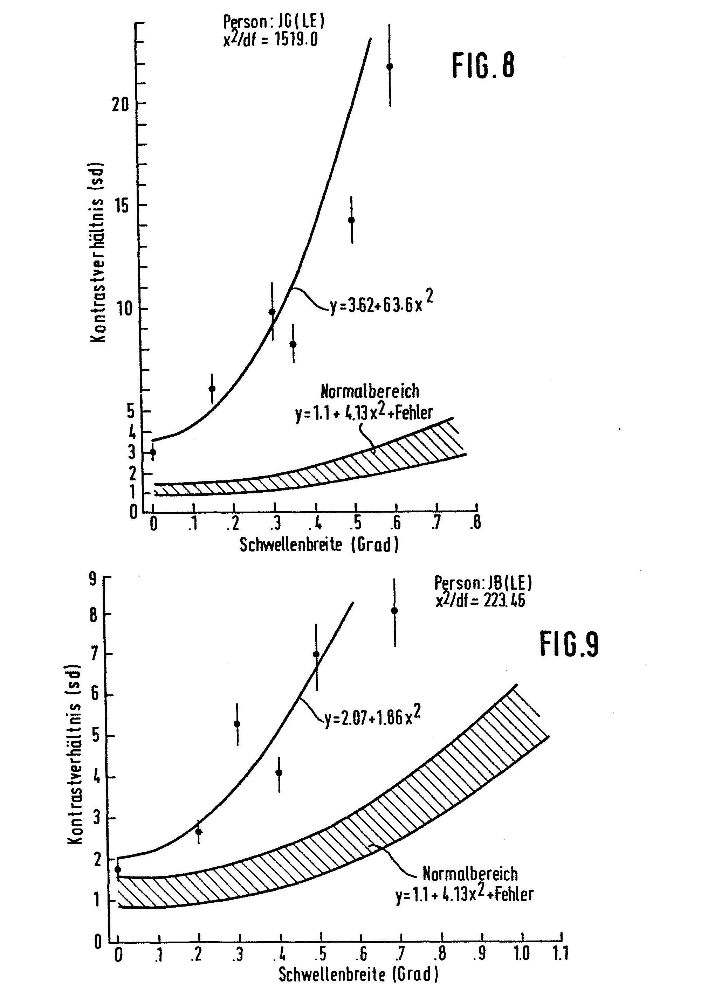

Die Fig. 8 bis 10 zeigen die Ergebnisse des
"Edgetest", der bei Versuchspersonen mit bestimmten Augenkrankheiten durchgeführt wurde. Die drei getesteten Patienten waren seit ihrer Kindheit insulinabhängig
und hatten eine normale Sehschärfe, d.h. 20/20 oder besser in jedem Auge.

Die Fig. 8 zeigt die Ergebnisse eines "Edgetests", der am linken Auge eines der Versuchspersonen
durchgeführt wurde. Die Sollwerte der Kontrollpersonen
liegen im schraffierten Bereich der Abbildung 8. Die
Daten zeigen, dass bei dieser Versuchsperson ein grösserer Kontrast zur Wahrnehmung einer Kante, und zwar bei allen Schwellenbreiten, als bei den Kontroll-Versuchspersonen erforderlich war. Folglich war die Kontrastempfindlichkeit bei dieser Versuchsperson ernstlich eingeschränkt.

Ein wichtiges methodisches Merkmal der vorliegenden Erfindung ist die Möglichkeit, die spezifische Grösse des Unterschiedes zwischen der Kontrastempfindlichkeit
der getesteten Versuchsperson und der Norm zu quantifizieren. Es wurde eine Bezugsnummer vorgegeben, um die
Ergebnisse einer jeden Funktion durch Bestimmung des
Wertes der Chi-Quadrat-Verteilung pro Freiheitsgrad
vergleichen zu können.

Es ist natürlich leicht einzusehen, dass oben
erwähnte Methode zur Ableitung einer Bezugszahl ein möglicher Algorithmus ist, der zur Quantifizierung der
Testergebnisse verwendet werden kann. Andere Algorithmen können auch verwendet werden, ohne dass Sinn und
Zweck der vorliegenden Erfindung überschritten werden.

Der Vorteil einer solchen Bezugszahl liegt
darin, dass sie auf einer Funktion und nicht auf einer
Punktabschätzung beruht und dem Augenarzt/Optiker eine
bestimmte Bezugszahl zur Verwendung in einer nachfolgenden Diagnose zur Verfügung stellt. Die Bezugszahl für
einen überprüften Normal-Beobachter würde etwa 5 $\pm$ 3
betragen. Die in der Fig. 8 getestete Versuchsperson
hatte eine ·Bezugszahl von 1519,0, die trotz einer normalen Sehschärfe auf einen schweren Verlust der Kontrast- empfindlichkeit hinweist.

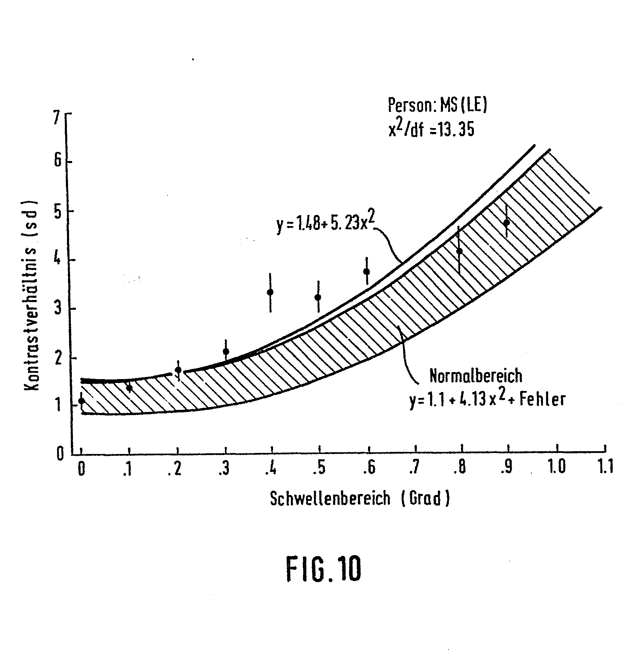

Die Ergebnisse der "Edgetests" in den Fig. 9 und 10 zeigen weniger schwere Netzhauterkrankungen an, worauf die Bezugszahl 223,46 der Versuchsperson in der Fig. 9 und 13,35 der Versuchsperson in der Fig. 10 hinweist. Die in der Fig. 9 getestete Versuchsperson wäre anfällig für eine Netzhauterkrankung, während die Testergebnisse für die in der Fig. 10 getestete Versuchsperson eine Krankheit vermuten lassen.

Die folgenden Beispiele sollen die Leistungsfähigkeit des "Edgetests" weiter verdeutlichen, wenn sie als Diagnosemittel bei Versuchspersonen verwendet werden, die zwar über einen Verlust ihrer Sehfähigkeit klagen, aber keine Erkrankung der Augen vorliegt.

Beispiel 1

Der "Edgetest" nach der vorliegenden Erfindung wurde mit einer Versuchsperson durchgeführt, die über einen Verlust der Sehfähigkeit im linken Auge klagte. Die Sehschärfe im linken Auge betrug 20/40 und die Testergebnisse der Gesichtsfeldmessung waren normal.

Es ergaben sich folgende "Edgetest"-Ergebnisse: Rechtes Auge 15,61, linkes Auge 22,63. Diese Testergebnisse weisen auf einen erkennbaren Unterschied zwischen den beiden Augen hin, wobei beide Werte einen mässigen Verlust der Kontrastempfindlichkeit anzeigen. Die Ergebnisse lassen erste Krankheitsanzeigen im linken Auge vermuten und bestätigen die Klagen der Versuchsperson über
eine verminderte Sehfähigkeit.


Beispiel 2

Eine Versuchsperson wurde mit "Edgetest"- geprüft, als sie sich darüber beklagte, dass zeitweilig
ein kleines verschwommenes Quadrat im linken Auge auftrat.
Die Sehstärke betrug 20/15 (normal) und die Ergebnisse
der Gesichtsfeldmessung waren normal. Die "Edgetest"-Ergebnisse lauteten wie folgt: Rechtes Auge 3,12, linkes Auge
19,94.

Die Ergebnisse des "Edgetestes" zeigen einen
Unterschied zwischen beiden Augen. Während die "Edgetest"-
Ergebnisse für das rechte Auge innerhalb der normalen Grenzen lagen, zeigten, die Ergebnisse für das linke Auge
einen mässigen Verlust der Kontrastempfindlichkeit. Der
"Edgetest" bestätigte in quantifizierbarer Form die Klagen der Versuchsperson. Anschliessende Tests wurden auf der
Grundlage dieser Ergebnisse fortgeführt.


Beispiel 3

Eine Versuchsperson wurde unter Verwendung
des "Edgetests" geprüft, als sie sich über eine plötzlich  auftretende und vier Wochen lang anhaltende Trübung
im linken Auge beklagte. Die Sehschärfe beider Augen sowie
die Ergebnisse der Gesichtsfeldmessung waren normal. Der "Edgetest" ergab folgende Ergebnisse: Linkes Auge 570,5, rechtes Auge 138,7.

Die Ergebnisse des "Edgetests" bestätigen einen deutlichen Verlust der Kontrastempfindlichkeit in beiden Augen. Das linke Auge zeigte eine starke Sehbeeinträchtigung und stimmte mit den Klagen der Versuchsperson über eine Trübung dieses Auges überein.

## Beispiel 4

Eine Versuchsperson wurde nach der vorläufigen Diagnose einer Krankheit bei verminderter Sehschärfe am linken Auge geprüft. Die gemessene Sehschärfe im linken Auge betrug 20/50. Eine Gesichtsfeldmessung wurde nicht vorgenommen. Der "Edgetest" zeigte folgende Ergebnisse: Linkes Auge 1847, rechtes Auge 34. Diese Werte zeigten einen substantiellen Verlust der Kontrastempfindlichkeit im linken Auge an, während eine Prüfung des rechten Auges nur einen minimalen Verlust vermuten liess. Die Ergebnisse des "Edgetests" wurden bestätigt und stimmten quantitativ mit den Klagen der Versuchsperson überein.

- 1 -

Patentansprüche

_____ _____ ___ __

1. Verfahren zum Prüfen der visuellen Wahrnehmungsfähigkeit einer Versuchsperson, für eine Schwelle zwischen benachbarten Feldern unterschiedlicher Leuchtdichte, dadurch gekennzeichnet, dass der Versuchsperson mehrere visuelle,wahrnehmbare Testmuster dargeboten werden, wobei diese Muster aus zumindest zwei benachbarten Feldern mit unterschiedlicher Leuchtdichte bestehen, die durch eine Uebergangsschwelle miteinander verbunden sind, und dass bei jedem der genannten Muster die wahrnehmbaren Schwellen festgestellt und festgehalten werden.

2. Verfahren nach Anspruch 1, dadurch gekennzeichnet, dass die Breite der Schwelle von einem Testmuster zu einem anderen variiert wird.

3. Verfahren nach Anspruch 2, dadurch gekennzeichnet, dass die Testmuster von einem Computer gewählt und der Versuchsperson elektronisch auf einer Bildfläche dargestellt werden.

4. Verfahren nach Anspruch 3, dadurch gekennzeichnet, dass die Leuchtdichte eines Feldes konstant gehalten und die Leuchtdichte eines anderen Feldes variiert wird.

5. Verfahren nach Anspruch 4, dadurch gekennzeichnet, dass die Leuchtdichte des variablen Feldes vom Computer vorgewählt wird.

6. Verfahren nach Anspruch 4, dadurch gekennzeichnet, dass die Leuchtdichte des variablen Feldes von der Versuchsperson über eine entsprechende Regelung eingestellt wird.

7. Verfahren nach Anspruch 5, dadurch gekennzeichnet, dass die Breite der Schwelle und der Unterschied in der Leuchtdichte zwischen den Feldern bei jedem Testmuster vom Computer vorgewählt und die visuelle Wahrnehmungsfähigkeit der Schwelle durch die Versuchsperson bestimmt wird.

8. Verfahren nach Anspruch 6, dadurch gekennzeichnet, dass die Schwellenbreite eines jeden Testmusters und die anfängliche Differenz in der Leuchtdichte zwischen den Feldern vom Computer vorgewählt wird und dass man die Versuchsperson die Leuchtdichte des variablen Feldes so lange variieren lässt bis sie die Schwelle nicht mehr sieht und diesen Schwellwert bestimmt.

9. Verfahren nach Anspruch 1, dadurch gekennzeichnet, dass fortgesetzte Prüfungen durchgeführt und die Prüfungsergebnisse im Vergleich mit Kontrollwerten ziffernmässig festgestellt werden, und dass Veränderungen der Ziffern als Veränderungen der Sehfähigkeit festgestellt werden.

10. Verfahren nach Anspruch 1, dadurch gekennzeichnet, dass die Muster auf eine Bildfläche mittels eines Projektionsgerätes projiziert werden.

11. Verfahren nach Anspruch 1, dadurch gekennzeichnet, dass wiederholt in Intervallen bis zu mehreren Wochen die visuelle Wahrnehmungsfähigkeit der Versuchsperson festgestellt und mit derjenigen einer Kontrollgruppe verglichen wird.

12. Vorrichtung zur Durchführung des Verfahrens nach Anspruch 1, gekennzeichnet durch Mittel zum Darbieten von Testmustern mit mindestens zwei benachbarten Feldern unterschiedlicher Leuchtdichte, die eine bestimmte Uebergangsschwelle aufweisen und durch Mittel zur Bestimmung der Fähigkeit der Versuchsperson, diese Schwelle zu erkennen.

13. Vorrichtung nach Anspruch 12, dadurch gekennzeichnet, dass die Mittel zum Darbieten einen Video-Monitor aufweisen.

14. Vorrichtung nach Anspruch 13, dadurch gekennzeichnet, dass bei den Mustern die Leuchtdichte eines der Felder konstant und die Leuchtdichte des anderen variabel ist.

15. Vorrichtung nach Anspruch 12, dadurch gekennzeichnet, dass die Mittel zum Darbieten einen Projektor aufweisen.

16. Vorrichtung nach Anspruch 12, dadurch gekennzeichnet, dass weiter Mittel zur Auswahl von Testmustern vorhanden sind.

17. Vorrichtung nach Anspruch 12, dadurch gekennzeichnet, dass weiter Mittel zur Steuerung der Schwellenbreite für jedes Muster vorhanden sind.

18. Vorrichtung nach Anspruch 14, dadurch gekennzeichnet, dass eine von der Versuchsperson betätigbare Regelung, für das Verstellen der Leuchtdichte des variablen Feldes vorhanden ist.

0096325

FIG.1

Standard Bedingungen (JH)
0,.75,0

FIG.2

FIG.3A

Schwellenbreite 0 Grad

Leuchtdichte

Probenbreite (Grad)

FIG.3B

Schwellenbreite 10 Grad

Leuchtdichte

Probenbreite (Grad)

FIG.3C

Schwellenbreite 66 Grad

Leuchtdichte

Probenbreite (Grad)

FIG. 4

$y = 1.1 + 4.13x^2$

FIG. 5

FIG. 6

Floppy Disk — 55

Computer — 10

Teletype — 60

Bus Steuerung — 20

Daten Bus

Patienten Antwortmodul — 30

Patienten Antworttastatur — 31

Bus Abschluss-modul

15

Speicher Modul — 35

Abfrage Generator Modul — 40

Analog Funktionsmodul — 45

Stimulus Darsteller — 50

X Y Z

4 / 7

0096325

FIG.7

Person: JG(LE)
$x^2/df = 1519.0$

FIG. 8

$y = 3.62 + 63.6x^2$

Normalbereich
$y = 1.1 + 4.13x^2 + Fehler$

Kontrastverhältnis (sd)

Schwellenbreite (Grad)

Person: JB(LE)
$x^2/df = 223.46$

FIG. 9

$y = 2.07 + 1.86x^2$

Normalbereich
$y = 1.1 + 4.13x^2 + Fehler$

Kontrastverhältnis (sd)

Schwellenbreite (Grad)

FIG.10